# EUROPEAN PATENT APPLICATION

(11) **EP 1 103 250 A1**
(43) Date of publication of application: **30.05.2001**
(21) Application number: 00124836.8
(22) Date of filing: 14.11.2000
(51) Int. Cl.: A61K 7/48

(54) **Oil-in-polyhydric alcohol type warming base agent comprising sucrose fatty acid ester**

(30) Priority: 15.11.1999 JP 32468099
(71) Applicant: SHISEIDO COMPANY LIMITED, Chuo-ku, Tokyo 104-8010 (JP)
(72) Inventor: Ide, Nobuyuki, c/o Shiseido Research Center, Yokohama-shi, Kanagawa 224-8558 (JP); Ishiwatari, Masaaki, c/o Shiseido Research Center, Yokohama-shi, Kanagawa 224-8558 (JP)
(74) Representative: TER MEER STEINMEISTER & PARTNER GbR

(57) **Abstract**

The present invention is an oil-in-polyhydric-alcohol type warming base agent containing the following ingredients:
1. Polyhydric alcohol: : 20.0-99.0 wt% of the total amount
2. Sucrose fatty acid ester: 0.5-10.0 wt% of the total amount
3. Oil: 30.0-80.0 wt% of the total amount

The object of the present invention is to provide an oil-in-polyhydric-alcohol type warming base agent which is superior in terms of its make-up removal effect, safety (low irritation), stability, and warming sensation.

## Description

### Related Application

This application claims the priority of Japanese Patent application No. 11-324680 filed on November 15, 1999, which is incorporated herein by reference.

### Background of the Invention

### 1. Field of the invention

The present invention invention relates to an oil-in-polyhydric-alcohol type warming base agent. More specifically, the present invention relates to the base agent of an endermic liniment which gives a make-up removal effect and a warming sensation. The warming base agent of the present invention is excellent in terms of safety and stability.

### 2. The Prior Art

Cosmetics which give a warming sensation are obtained by a high blend ratio of a humectant such as glycerin. These are widely used mainly as body care products for massaging. However, there are problems as follows:
1. In view of irritation, a warming base agent having a large amount of a humectant cannot be used on the face.
2. Although blending polyethylene glycol into a warming base agent gives a superior warming sensation, this has a shortcoming in terms of worsened stability of the base agent.

The object of the present invention is as follows:
1. To provide a warming base agent that gives both a warming sensation and a make-up removal effect.
2. To provide a warming base agent with less irritation.
3. To provide a warming base agent that is safe and stable.

### Brief Summary of the Invention

The inventors conducted earnest research to solve the aforementioned problems and completed the present invention. The present invention is as follows.
1. An oil-in-polyhydric-alcohol type warming base agent comprising the following ingredients:
   (1) Polyhydric alcohol: 20.0-99.0 wt% of the total amount
   (2) Sucrose fatty acid ester: 0.5-10.0 wt% of the total amount
   (3) Oil: 30.0-80.0 wt% of the total amount
2. Said oil-in-polyhydric-alcohol type warming base agent wherein said polyhydric alcohol is at least one compound selected from the group consisting of the following: glycerin, diglycerin, polyglycerin, 1,3-butylene glycol, dipropylene glycol, polyethylene glycol, trimethlolethane, trimethlolpropane, erythritol, pentaerythritol, sorbitan, glucose, sorbitol, maltitol, sucrose, raffinose, and trehalose.
3. Said oil-in-polyhydric-alcohol type warming base agent wherein said polyhydric alcohol is polyethylene glycol.
4. Said oil-in-polyhydric-alcohol type warming base agent wherein said sucrose fatty acid ester meets the following conditions:
   (1) 50 wt% or more of the fatty acid which constitutes the sucrose fatty acid ester is a saturated fatty acid having 8-18 carbon atoms or an unsaturated fatty acid having 16-22 carbon atoms.
   (2) 75 wt% or more of the sucrose fatty acid ester is monoester.

### Detailed Description of the Invention

The present invention is described in detail is follows.

### (1) Polyhydric alcohol

Examples of the polyhydric alcohol used in the present invention include, but are not Imited to: glycerin, diglycerin, polyglycerin, 1,3-butylene glycol, dipropylene glycol, polyethylene glycol, trimethlolpropane, erythritol, pentaerythritol, sorbitan, glucose, sorbitol, maltitol, sucrose, raffinose, and trehalose. One or a combination of two or more of these can be used. Of these, the preferable polyhydric alcohols are as follows: glycerin, 1,3-butylene glycol, dipropylene glycol, and polyethylene glycol. Polyethylene glycol is particularly preferable. When two or more polyhydric alcohols are used in combination, the polyethylene glycol content in the total amount of the polyhydric alcohol is preferably 20.0 wt% or higher.

The blend ratio of the polyhydric alcohol is 20.0-99.0 wt% of the total amout of the warming base agent. A preferable range is 20.0-50.0 wt%. If the blend ratio is less than 20.0 wt%, then the warming sensation due to the polyhydric alcohol is not sufficient. If the blend ratio is more than 99.0 wt%, then irritation may result when applied on the face of a person who has sensitive skin.

### (2) Sucrose fatty acid ester

The sucrose fatty acid ester used in the present invention should preferably meet the following conditions:
1. 50 wt% or more of the fatty acid which constitutes the sucrose fatty acid is a saturated fatty acid having 8-18 carbon atoms or an unsaturated fatty acid having 16-22 carbon atoms.
2. 75 wt% or more of the sucrose fatty acid ester is a monoester.

The blend ratio of the monoester is preferably 75 wt% or more, more preferably 97 wt% or more, and most preferably 100 wt%.

Specific examples of the sucrose fatty acid ester that meet the aforementioned conditions are as follows: sucrose caprylate, sucrose caprate, sucrose laurate, sucrose myristate, sucrose palmitate, sucrose stearate, sucrose oleate, and sucrose erucate. One or a combination of two or more of these are used. Of these, sucrose oleate and sucrose erucate are preferable.

The blend ratio of the sucrose fatty acid ester is typically 0.5-10.0 wt%, preferably 1.0-10.0 wt%, of the total amount of the warming base agent. If the blend ratio is less than 0.5 wt%, then the stability over time becomes poor when a high blend ratio of oil is added to the gel-like composition obtained from the polyhydric alcohol and the sucrose fatty acid ester. If the blend ratio is more than 10.0 wt%, then a hard gel-like composition results. As a result, during use, the warming base agent tends to (1) lose its ease of spreading and (2) gives rise to annoying stickiness.

### (3) Oil

Examples of the oil used in the present invention are as follows:
hydrocarbons that are solid or liquid at ordinary temperatures (20°C), higher alcohol higher fatty acid monoester compounds, dibasic acid higher alcohol diester compounds, glycol higher fatty acid diester compounds, animal/plant oils, and synthesized triglyceride compounds. One or a combination of two or more of these are used.

The blend ratio of the oil is typically 30.0-80.0 wt%, preferably 40.0-70.0 wt%, of the total amount of the warming base agent. If the blend ratio is less than 30.0 wt% then the blend ratio of the polyhydric alcohol must be increased to obtain a warming sensation, which may result in irritation. If the blend ratio is more than 80.0 wt%, then the stability of the warming base agent becomes poor, which can result in phase separation.

In the present invention, common ingredients of endermic liniments can be added as necessary in addition to the aforementioned essential ingredients, and the target product can be manufactured using a conventional method.

Examples of these common ingredients are as follows: pigments, perfumes, ultraviolet absorbents agents, antiseptics, drugs, chelating agents, polymers, cleaning agents and surfactants.

The effects of the oil-in-polyhydric-alcohol type warming base agent of this invention are as follows:
1. It has an excellent warming sensation.
2. Irritation can be suppressed. With a high blend ratio of the oil, skin irritation by the polyhydric alcohol can be suppressed. Therefore an excellent skin care effect is achieved.
3. It can be used preferably as a warming base agent with a make-up removal effect.

The effects of the present invention are as follows.
1. An oil-in-polyhydric-alcohol type warming base agent which is superior in terms of its make-up removal effect, safety (low irritation), stability, and warming sensation can be provided.

### Examples

Further details of the present invention are described below by referring to Examples. Blend ratios are expressed in weight percent units unless specified otherwise.

### Examples 1-4, Comparative examples 1-3

Using the formulas shown in Table 1, make-up removal cosmetics (samples) using the oil-in-polyhydric-alcohol type warming base agent were prepared with a conventional method. Using these, the following items were evaluated. The results are shown in Table 1.
(1) Warming sensation during use
(2) Ease of spreading during use
(3) Make-up removal effect
(4) Irritation
(5) Stability

The evaluation methods are as described below.
(1) Warming sensation during use
   The make-up removal cosmetics were applied on the faces of 30 female panelists for evaluation. A questionnaire survey was conducted and the results were judged using the following criteria.
   ⓞ(excellent): 20 or more panelists reported a strong warming sensation.
   ○ (good) 10-19 panelists reported a strong warming sensation.
   Δ (fair): 5-9 panelists reported a strong warming sensation.
   × (poor): 4 or less panelists reported a strong warming sensation.
(2) Ease of spreading during use
   The make-up removal cosmetics were applied on the faces of 30 female panelists for evaluation. A questionnaire survey was conducted and the results were judged using the following criteria.
   ⓞ (excellent): 20 or more panelists reported very easy spreading.
   O (good): 10-19 panelists reported very easy spreading.
   Δ (fair): 5-9 panelists reported very easy spreading.
   × (poor): 4 panelists reported very easy spreading.
(3) Make-up removal effect
   The make-up removal cosmetics were applied on the faces of 30 female panelists for evaluation. A questionnaire survey was conducted and the results were judged using the following criteria.
   ⓞ (excellent): 20 or more panelists reported that the make-up removal effect was very high.
   O (good) : 10-19 panelists reported that the make-up removal effect was very high.
   Δ (fair): 5-9 panelists reported that the make-up removal effect was very high.
   × (poor): 4 panelists reported that the make-up removal effect was very high.
(4) Irritation
   The make-up removal cosmetics were applied on the faces of 30 female panelists for evaluation. A questionnaire survey was conducted and the results were judged using the following criteria.
   ⓞ (excellent): 20 or more panelists reported no irritation.
   ○ (good): 10-19 panelists reported no irritation.
   Δ (fair): 5-9 panelists reported no irritation.
   × (poor): 4 panelists reported strong no irritation.
(5) Stability
   The make-up removal cosmetics were allowed to stand for one month at room temperature (25°C). The appearance was visually evaluated using the following criteria.
   ○ : No changes in appearance were observed, indicating excellent stability.
   Δ : Some oil separation was observed on the surface.
   × : The oil was separated and two layers were observed.

**Table 1**

| | Example | | | Comparative example | | | |
|---|---|---|---|---|---|---|---|
| | 1 | 2 | 3 | 4 | 1 | 2 | 3 |
| Sorbitol | 20.0 | 5.0 | 10.0 | 5.0 | 5.0 | 10.0 | 5.0 |
| 1,3-butylene | 2.0 | 3.0 | 5.0 | 3.0 | 2.0 | 5.0 | 3.0 |
| glycol | | | | | | | |
| Glycerin | 10.0 | 20.0 | 20.0 | 10.0 | 5.0 | 20.0 | 10.0 |
| 5.0 Polyethylene | 10.0 | 20.0 | 20.0 | 20.0 | 5.0 | 20.0 | 10.0 |
| glycol | | | | | | | |
| Sucrose stearate | 3.0 | 3.0 | 3.0 | 3.0 | 3.0 | 0.2 | 12.0 |
| Purified water | Balance | Balance | Balance | Balance | Balance | Balance | Balance |
| Sodium edetate | 0.05 | 0.05 | 0.05 | 0.05 | 0.05 | 0.05 | 0.05 |
| PEG/PPG copolymer PEG/PPG copolymer | - | - | - | 3.0 | - | - | - |
| Liquid paraffin | 50.0 | 50.0 | 35.0 | 50.0 | 70.0 | 35.0 | 50.0 |
| Petrolatum | 2.0 | 2.0 | 2.0 | 2.0 | 2.0 | 2.0 | 2.0 |
| Vitamin E | 0.05 | 0.05 | 0.05 | 0.05 | 0.05 | 0.05 | 0.05 |
| derivative | | | | | | | |
| Perfume | Appropriate amount | Appropriate amount | Appropriate amount | Appropriate amount | Appropriate amount | Appropriate amount | Appropriate amount |
| Warming sensation | ○ | ⓞ | ⓞ | ⓞ | Δ | ⓞ | ○ |
| Ease of spreading | ○ | ○ | ○ | ○ | ○ | ⓞ | × |
| Make-up removal | ○ | ○ | ○ | ⓞ | ○ | ○ | ○ |
| effect | | | | | | | |
| Irritation | ○ | ○ | Δ | ○ | ⓞ | Δ | ○ |
| Stability | ○ | ○ | Δ | ○ | ○ | × | ○ |

The table shown above shows the excellent effects of Examples of the present invention. Other Examples of the present invention are described below.

Example 5: A make-up removal cosmetic with a warming sensation

| (A) | |
|---|---|
| Sorbitol | 5.0 wt% |
| 1,3-butylene glycol | 3.0 |
| Glycerin | 10.0 |
| Polyethylene glycol | 20.0 |

| (B) | |
|---|---|
| Sucrose myristate | 5.0 |

| (C) | |
|---|---|
| Purified water | Balance |
| Sodium edetate | 0.05 |
| PEG/PPG copolymer | 5.0 |

| (D) | |
|---|---|
| Liquid paraffin | 45.0 |
| Petrolatum | 4.0 |
| Vitamin E derivative | 0.02 |
| Perfume | Appropriate amount |

### (Preparation method)

Preparation was conducted according to the following procedure.
1) (A) phase is mixed homogeneously and heated up to 60°C.
2) (B) is added to this and mixed/dispersed homogeneously.
3) (C), which has been dissolved and mixed beforehand, is added to this and mixed homogeneously.
4) (D), which has been heated/dissolved at 60°C beforehand, is gradually added to this while homogeneously emulsified/dispersed.

### Example 6: A massaging base agent with a warming sensation

| (A) | |
|---|---|
| Sorbitol | 5.0 wt% |
| Glycerin | 20.0 |
| Polyethylene glycol | 20.0 |

| (B) | |
|---|---|
| Sucrose oleate | 3.0 |

| (C) | |
|---|---|
| Purified water | Balance |
| Sodium edetate | 0.05 |

| (D) | |
|---|---|
| Liquid paraffin | 40.0 |
| Petrolatum | 7.0 |
| Vitamin E derivative | 0.02 |
| Perfume | Appropriate amount |

| (E) | |
|---|---|
| Spherical polyethylene powder | 3.0 |

### (Preparation method)

Preparation was conducted according to the following procedure.
1) (A) phase is mixed homogeneously and heated up to 60°C.
2) (B) is added to this and mixed/dispersed homogeneously.
3) (C), which has been dissolved and mixed beforehand, is added to this and mixed homogeneously.
4) (D), which has been heated/dissolved at 60°C beforehand, is gradually added to this, while homogeneously emulsified/dispersed.
5) Finally, (E) is added to this and stirred/mixed homogeneously.

### Example 7: A body cleaning base agent with a warming sensation

| (A) | |
|---|---|
| 1,3-butylene glycol | 5.0 wt% |
| Glycerin | 5.0 |
| Polyethylene glycol | 25.0 |

| (B) | |
|---|---|
| Sucrose stearate | 5.0 |

| (C) | |
|---|---|
| Purified water | Balance |
| Sodium edetate | 0.05 |
| Cocoyl propyldimethyl glycine | 2.0 |

| (D) | |
|---|---|
| Liquid paraffin | 40.0 |
| Petrolatum | 2.0 |
| Vitamin E derivative | 0.02 |
| Perfume | Appropriate amount |

| (E) | |
|---|---|
| Spherical polyethylene powder | 5.0 |
| Nylon powder | 10.0 |

### (Preparation method)

Preparation was conducted according to the following procedure.
1) (A) phase is mixed homogeneously and heated up to 60°C.
2) (B) is added to this and mixed/dispersed homogeneously.
3) (C), which has been dissolved and mixed beforehand, is added to this and mixed homogeneously.
4) (D) which has been heated/dissolved at 60°C beforehand, is gradually added to this, while homogeneously emulsified/dispersed.
5) Finally, (E) is added to this and stirred/mixed homogeneously.

The warming base agents obtained in Examples 5-7 are stable, give a warming sensation, cause no irritation, and have a make-up removal effect.

## Claims

1. An oil-in-polyhydric-alcohol type warming base agent comprising the following ingredients:
1. Polyhydric alcohol: 20.0-99.0 wt% of the total amount
2. Sucrose fatty acid ester: 0.5-10.0 wt% of the total amount
3. Oil: 30.0-80.0 wt% of the total amount

2. The oil-in-polyhydric-alcohol type warming base agent of claim 1 wherein said polyhydric alcohol is at least one compound selected from the group consisting of the following: glycerin, diglycerin, polyglycerin, 1,3-butylene glycol, dipropylene glycol, polyethylene glycol, trimethlolethane, trimethlolpropane, erythritol, pentaerythritol, sorbitan, glucose, sorbitol, maltitol, sucrose, raffinose, and trehalose.

3. The oil-in-polyhydric-alcohol type warming base agent of claim 1 wherein said polyhydric alcohol is polyethylene glycol.

4. The oil-in-polyhydric-alcohol type warming base agent of claim 1, 2, or 3 wherein said sucrose fatty acid ester meets the following conditions:
1. 50 wt% or more of the fatty acid which constitutes the sucrose fatty acid ester is a saturated fatty acid having 8-18 carbon atoms or an unsaturated fatty acid having 16-22 carbon atoms.
2. 75 wt% or more of the sucrose fatty acid ester is monoester.
